(19) Europäisches Patentamt / European Patent Office / Office européen des brevets

(11) **EP 4 147 635 B1**

(12) **EUROPEAN PATENT SPECIFICATION**

(45) Date of publication and mention
of the grant of the patent:
**01.01.2025 Bulletin 2025/01**

(21) Application number: **22194381.4**

(22) Date of filing: **07.09.2022**

(51) International Patent Classification (IPC):
*A61B 5/0507* (2021.01)    *A61B 5/08* (2006.01)
*A61B 5/113* (2006.01)    *G16H 50/20* (2018.01)
*G16H 50/30* (2018.01)

(52) Cooperative Patent Classification (CPC):
**A61B 5/0022; A61B 5/0507; A61B 5/0826;
A61B 5/113; A61B 5/4088; A61B 5/4818;
A61B 5/7264; A61B 5/7282; G16H 50/20;
G16H 50/30**

(54) **DEVICE, METHOD AND COMPUTER PROGRAM FOR ANALYZING SLEEP BREATHING USING RADAR**

VORRICHTUNG, VERFAHREN UND COMPUTERPROGRAMM ZUR ANALYSE VON SCHLAFATMUNG MIT RADAR

DISPOSITIF, PROCÉDÉ ET PROGRAMME INFORMATIQUE POUR ANALYSER LA RESPIRATION PENDANT LE SOMMEIL À L'AIDE D'UN RADAR

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.09.2021 KR 20210120587
09.12.2021 KR 20210176121**

(43) Date of publication of application:
**15.03.2023 Bulletin 2023/11**

(73) Proprietor: **BITSENSING INC.**
**Seoul 04778 (KR)**

(72) Inventor: **CHOE, Sun Taag**
**07013 Seoul (KR)**

(74) Representative: **BCKIP Part mbB**
**Siegfriedstraße 8**
**80803 München (DE)**

(56) References cited:
**CN-A- 112 263 242    KR-A- 20200 071 013**

• **WANG QISONG ET AL: "Frequency-Modulated Continuous Wave Radar Respiratory Pattern Detection Technology Based on Multifeature", JOURNAL OF HEALTHCARE ENGINEERING, vol. 2021, 10 August 2021 (2021-08-10), Brentwood, pages 1 - 18, XP093011102, ISSN: 2040-2295, Retrieved from the Internet <URL:https://downloads.hindawi.com/journals/jhe/2021/9376662.pdf> DOI: 10.1155/2021/9376662**
• **KAGAWA MASAYUKI ET AL: "Non-contact diagnostic system for sleep apnea-hypopnea syndrome based on amplitude and phase analysis of thoracic and abdominal Doppler radars", MEDICAL AND BIOLOGICAL ENGINEERING AND COMPUTING, SPRINGER, HEILDELBERG, DE, vol. 54, no. 5, 26 August 2015 (2015-08-26), pages 789 - 798, XP035941313, ISSN: 0140-0118, [retrieved on 20150826], DOI: 10.1007/S11517-015-1370-Z**

## Description

### TECHNICAL FIELD

The present disclosure relates to a device, a method and a computer program for analyzing sleep breathing using a radar.

### BACKGROUND

Polysomnography is a test used to measure the quality and amount of sleep and detect sleep diseases and sleep-related disorders. In general, polysomnography is used to detect a variety of sleep disorders by measuring physiological and physical signals from human body during sleep. For example, brain waves, eletrooculogram, electromyogram, electrocardiogram, arterial blood, oxygen saturation, abdominal and thoracic breathing exercises, respiratory air flow, snoring and body postures are measured.

A basic method for measuring the quality and amount of sleep uses a wrist actigraphy device to measure sleep time. Specifically, sleep time of a wearer wearing a wrist actigraphy device is measured and tossing or the like during sleep is detected based on activities of the wearer.

Also, a photoplethysmography (PPG) device worn on the wrist is used to measure the heart rate and heart rate variability of the wearer during sleep. Specifically, sleep stages of the wearer are distinguished, and oxygen desaturation caused by apnea is detected by measuring oxygen saturation (SpO2).

However, the conventional test method can predict the quality of sleep (for example, satisfaction caused by tiredness), but cannot distinguish between obstructive sleep apnea (OSA) and central sleep apnea (CSA) that cause apnea.

*Specifi*cally, sleep apnea is divided into OSA that is a repetitive breathing cessation during sleep due to obstruction of upper airway and CSA that is a pause in breathing during sleep without giving effort to breathe. OSA is a representative disease of sleep disorder, accounting for about 90% of sleep apnea cases, and CSA is observed only in some cases.

The conventional test method needs to be performed while a tester is worn on a part of a human body, and depends only on an expensive tester to more precisely examine sleep diseases.

(Patent Document 1) Korean Patent Publication No. 10-2321991 (registered on October 29, 2021)
(Patent Document 2) Korean Patent Laid-open Publication No. 10-2018-0077453 (published on July 9, 2018)
Radar-based breathing analysis according to the state of the art is for instance also described in WANG QISONG ET AL: "Frequency-Modulated Continuous Wave Radar Respiratory Pattern Detection Technology Based on Multifeature", JOURNAL OF HEALTHCARE ENGINEERING, vol. 2021, 10 August 2021 (2021-08-10), pages 1-18, ISSN: 2040-2295, DOI: 10.1155/2021/9376662.

### DISCLOSURE OF THE INVENTION

### PROBLEMS TO BE SOLVED BY THE INVENTION

In view of the foregoing, the present disclosure provides a device, a method and a computer program for analyzing sleep breathing that can classify breathing patterns of a human during sleep and can also detect sleep breathing disorders during sleep by using a radar.

The problems to be solved by the present disclosure are not limited to the above-described problems. There may be other problems to be solved by the present disclosure.

### MEANS FOR SOLVING THE PROBLEMS

According to an embodiment of the invention, there is provided a device for analyzing sleep breathing according to claim 1.

According to another embodiment of the invention, there is provided a method for analyzing sleep breathing according to claim 7.

According to another embodiment of the invention, there is provided a computer program for analyzing sleep breathing according to claim 13.

This summary is provided by way of illustration only and should not be construed as limiting in any manner.

## EFFECTS OF THE INVENTION

According to any one of the above-described embodiments of the present disclosure, it is possible to analyze sleep-related breathing signals during sleep of a human by using a radar and thus possible to calculate the number of arousals caused by breathing disorders during sleep. It is possible to detect sleep disorders and determine sleep-related diseases by using the breathing signals from the human.

Also, the radar may be used to determine sleep-related disease including OSA, CSA and mixed sleep apnea. That is, it is possible to precisely determine sleep-related disease of the human and analyze the causes thereof as in polysomnography without performing polysomnography.

Further, the radar can be used to easily analyze sleep breathing of the human in daily life. Therefore, it is possible to continuously monitor sleep-related disease. Furthermore, it is possible to provide a device, a method and a computer program for analyzing sleep breathing that can be easily used in a contactless manner.

## BRIEF DESCRIPTION OF THE DRAWINGS

FIG. 1 is a configuration diagram of a sleep breathing analysis system according to an embodiment of the present disclosure.

FIG. 2 is a configuration diagram of a sleep breathing analysis device according to an embodiment of the present disclosure.

FIG. 3 is a diagram for explaining a process of calculating an average breathing signal according to an embodiment of the present disclosure.

FIG. 4 is a diagram for explaining a time sensitivity factor and an amplitude sensitivity factor according to an embodiment of the present disclosure.

FIG. 5 is a diagram for explaining first sleep breathing pattern information and second sleep breathing pattern information according to an embodiment of the present disclosure.

FIG. 6 is a diagram for explaining a first sleep breathing event and a second sleep breathing event according to an embodiment of the present disclosure.

FIG. 7A is a diagram for explaining optimal parameters for a plurality of sleep items according to an embodiment of the present disclosure.

FIG. 7B is a diagram showing an example of a data set for polysomnography according to an embodiment of the present disclosure.

FIG. 8 shows examples of distribution information of sleep-related disease of according to an embodiment of the present disclosure.

FIG. 9 is a diagram for explaining a process of determining whether a subject has sleep-related disease according to an embodiment of the present disclosure.

FIG. 10 is a flowchart showing a sleep breathing analysis method according to an embodiment of the present disclosure.

FIG. 11 is a flowchart showing a disease determination method according to an embodiment of the present disclosure.

## BEST MODE FOR CARRYING OUT THE INVENTION

Hereafter, example embodiments will be described in detail with reference to the accompanying drawings so that the present disclosure may be readily implemented by those skilled in the art. However, it is to be noted that the present disclosure is not limited to the example embodiments but can be embodied in various other ways. In the drawings, parts irrelevant to the description are omitted for the simplicity of explanation, and like reference numerals denote like parts through the whole document.

Throughout this document, the term "connected to" may be used to designate a connection or coupling of one element to another element and includes both an element being "directly connected" another element and an element being "electronically connected" to another element via another element. Further, it is to be understood that the terms "comprises," "includes," "comprising," and/or "including" means that one or more other components, steps, operations, and/or elements are not excluded from the described and recited systems, devices, apparatuses, and methods unless context dictates otherwise; and is not intended to preclude the possibility that one or more other components, steps, operations, parts, or combinations thereof may exist or may be added.

Throughout this document, the term "unit" may refer to a unit implemented by hardware, software, and/or a combination thereof. As examples only, one unit may be implemented by two or more pieces of hardware or two or more units may be implemented by one piece of hardware.

Throughout this document, a part of an operation or function described as being carried out by a terminal or device may be implemented or executed by a device connected to the terminal or device. Likewise, a part of an operation or function described as being implemented or executed by a device may be so implemented or executed by a terminal or device connected to the device.

Hereinafter, embodiments of the present disclosure will be explained in detail with reference to the accompanying drawings.

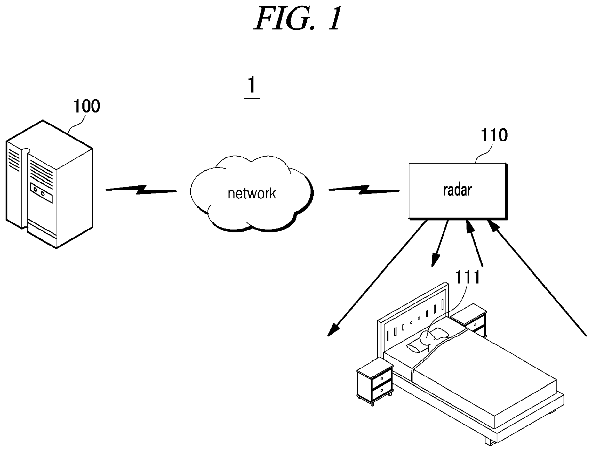

**FIG. 1** is a configuration diagram of a sleep breathing analysis system according to an embodiment of the present disclosure. Referring to **FIG. 1,** a sleep breathing analysis system 1 may include a sleep breathing analysis device 100 and a radar 110.

The components of the sleep breathing analysis system 1 illustrated in **FIG. 1** are typically connected to each other via a network. For example, as illustrated in **FIG. 1,** the sleep breathing analysis device 100 and the radar 110 may be connected simultaneously or sequentially.

The network refers to a connection structure that enables information exchange between nodes such as devices, devices, etc. and includes LAN (Local Area Network), WAN (Wide Area Network), Internet (WWW: World Wide Web), a wired or wireless data communication network, a telecommunication network, a wired or wireless television network, and the like. Examples of the wireless data communication network may include 3G, 4G, 5G, 3GPP (3rd Generation Partnership Project), LTE (Long Term Evolution), WIMAX (World Interoperability for Microwave Access), Wi-Fi, Bluetooth communication, infrared communication, ultrasonic communication, VLC (Visible Light Communication), LiFi, and the like, but may not be limited thereto.

The sleep breathing analysis device 100 may analyze breathing signals from a subject 111 by using the radar 110 during sleep of the subject 111. The sleep breathing analysis device 100 may detect a sleep disorder and determine a sleep-related disease by using the breathing signals from the subject 111.

For example, the sleep breathing analysis device 100 is arranged to keep a predetermined distance from the subject 111 in sleep, and may transmit a radar signal toward the subject 111 and receive the radar signal reflected from the subject 111 by using the radar 110.

The sleep breathing analysis device 100 may determine sleep-related disease, such as OSA, CSA and mixed sleep apnea, of the subject 111 by using the radar 110.

Therefore, the sleep breathing analysis device 100 can precisely determine sleep-related disease of the subject 111 and analyze the causes as in polysomnography without performing polysomnography.

Since the sleep breathing analysis device 100 analyzes the breathing signals related to the sleep of the subject 111 by using the radar 110, it is possible to reduce inconvenience incurred in performing polysomnography. Also, the sleep breathing analysis device 100 can easily analyze sleep breathing of the subject 111 in daily life and continuously monitor sleep-related disease. Further, the sleep breathing analysis device 100 can analyze sleep breathing of the subject 111 in a contactless manner.

Hereafter, each component of the sleep breathing analysis device 100 will be described.

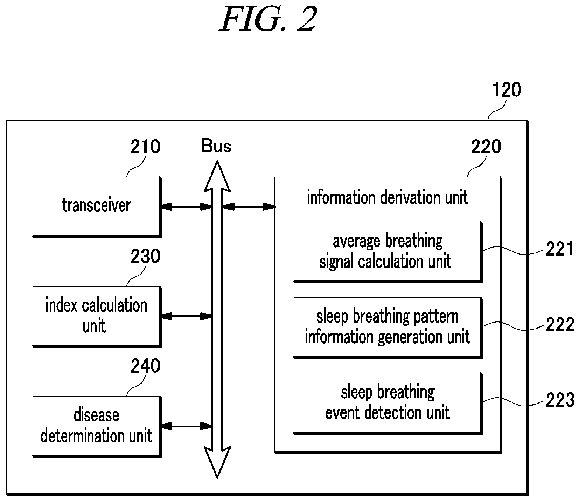

**FIG. 2** is a configuration diagram of a sleep breathing analysis device according to an embodiment of the present disclosure. Referring to **FIG. 2,** the sleep breathing analysis device 100 may include a transceiver 210, an information derivation unit 220, an index calculation unit 230 and a disease determination unit 240. However, these components 210 to 240 are just examples of components that can be controlled by the sleep breathing analysis device 100.

Hereafter, each component of the sleep breathing analysis device 100 will be described.

The transceiver 210 may transmit a radar signal toward a subject. The transceiver 210 may receive the radar signal reflected from the subject. For example, the transceiver 210 may transmit a radar signal toward a subject and receive the radar signal reflected from the subject by using a radar.

The information derivation unit 220 may calculate an average breathing signal of the subject by using the radar signal reflected from the subject, generate sleep breathing pattern information based on the average breathing signal, and detect a sleep breathing event occurring during sleep.

The information derivation unit 220 may include an average breathing signal calculation unit 221, a sleep breathing pattern information generation unit 222 and a sleep breathing event detection unit 223. The average breathing signal calculation unit 221 may calculate an average breathing signal of a subject based on a radar signal. For example, the average breathing signal calculation unit 221 may calculate a signal corresponding to usual breathing of the subject by using a pattern of the radar signal reflected from the subject and a change in amplitude of the signal.

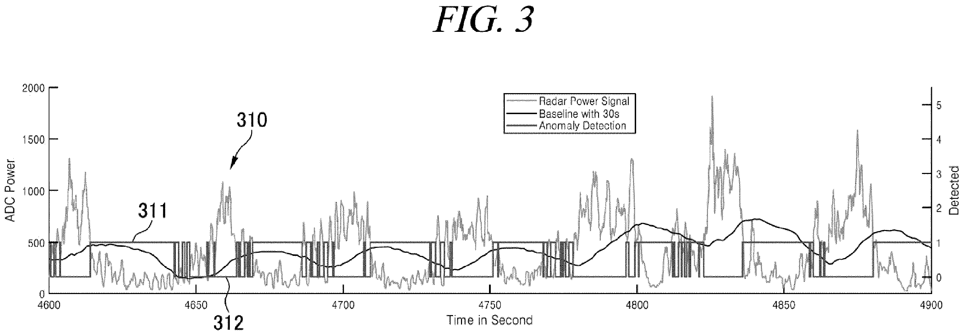

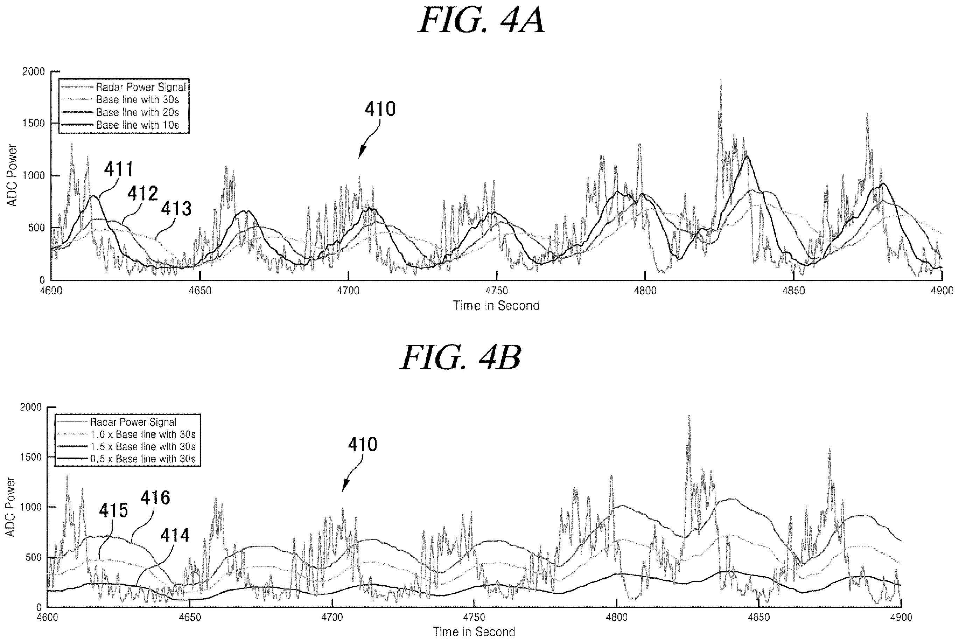

The average breathing signal calculation unit 221 may calculate the average breathing signal of the subject by using the radar signal reflected from the subject and two sensitivity factors. Hereafter, a process of calculating an average breathing signal will be described with reference to **FIG. 3** and **FIG. 4.**

**FIG. 3** is a diagram for explaining a process of calculating an average breathing signal according to an embodiment of the present disclosure. Referring to **FIG. 3,** the average breathing signal calculation unit 221 may calculate an average breathing signal 311 of the subject by using a radar signal 310 reflected from the subject and generate sleep breathing state information 312 of the subject.

For example, the average breathing signal calculation unit 221 may calculate the average breathing signal 311 by using a time sensitivity factor and an amplitude sensitivity factor based on the radar signal 310. The average breathing signal calculation unit 221 may use Equation 1 below.

<Equation 1>

$$m[i] = \frac{F}{L} \sum_{j=0}^{L-1} x[i-j]$$

In Equation 1, m is an average breathing signal of a subject and x is the radar signal reflected and received from the subject. Also, L is a time sensitivity factor that means the length of time between specific consecutive periods and is an integer obtained by multiplying the frequency of the radar signal in sequence by the length of time in second. Further, F is an amplitude sensitivity factor that responds to the amplitude between the specific consecutive periods.

For example, the average breathing signal calculation unit 221 may calculate the average breathing signal of the subject by using the time sensitivity factor and the amplitude sensitivity factor based on the radar signal. Referring to Equation 1, the waveform of the average breathing signal of the subject may become obtuse over time as the time sensitivity factor increases and the amplitude sensitivity factor decreases, and may become sharp over time as the time sensitivity factor decreases and the amplitude sensitivity factor increases.

The average breathing signal calculation unit 221 may compare the radar signal 310 received from the subject with the average breathing signal 311. For example, the average breathing signal calculation unit 221 may detect a section in which the radar signal 310 is smaller than the average breathing signal 311. The average breathing signal calculation unit 221 may detect the section in which the radar signal 310 is smaller than the average breathing signal 311 and generate the sleep breathing state information 312.

The average breathing signal calculation unit 221 may assign "1" to the section in which the radar signal 310 is smaller than the average breathing signal 311 and "0" to the other section. For example, the average breathing signal calculation unit 221 may generate breathing state information about sleep breathing of the subject based on the detection result. That is, the sleep breathing state information 312 of the subject may be expressed by a value of "1" or "0".

**FIG. 4** is a diagram for explaining a time sensitivity factor and an amplitude sensitivity factor according to an embodiment of the present disclosure. For example, the average breathing signal calculation unit 221 may calculate an average breathing signal of a subject based on a radar signal received from the subject. In this case, the average breathing signal calculation unit 221 may use the time sensitivity factor and the amplitude sensitivity factor.

**FIG. 4A** is a graph showing an average breathing signal depending on a change in time sensitivity factor and calculated from a radar signal 410, and **FIG. 4B** is a graph showing an average breathing signal depending on a change in amplitude sensitivity factor and calculated from the radar signal 410.

Referring to the example shown in **FIG. 4A** and Equation 1 above, as a cycle of a time sensitivity factor decreases, the waveform of an average breathing signal corresponding to the time sensitivity factor may become sharp. For example, as a result of comparison among a waveform 411 of an average breathing signal corresponding to a time sensitivity factor with a cycle of 10 seconds, a waveform of an average breathing signal corresponding to a time sensitivity factor 412 with a cycle of 20 seconds and a waveform of an average breathing signal corresponding to a time sensitivity factor 413 with a cycle of 30 seconds, the waveform 411 of the average breathing signal corresponding to the time sensitivity factor with a cycle of 10 seconds changes most sharply.

Referring to the example shown in **FIG. 4B** and Equation 1 above, as a cycle of an amplitude sensitivity factor decreases, the waveform of an average breathing signal corresponding to the amplitude sensitivity factor may become obtuse. For example, as a result of comparison among a waveform 414 of an average breathing signal corresponding to an amplitude sensitivity factor with a cycle of 0.5, a waveform of an average breathing signal corresponding to an amplitude sensitivity factor 415 with a cycle of 1.0 and a waveform of an average breathing signal corresponding to an amplitude sensitivity factor 416 with a cycle of 1.5, the waveform 414 of the average breathing signal corresponding to the amplitude sensitivity factor with a cycle of 0.5 can be most obtuse.

The sleep breathing pattern information generation unit 222 may compare a radar signal with an average breathing signal and generate sleep breathing pattern information of a subject. For example, the sleep breathing pattern information generation unit 222 may detect a section in which a radar signal received from a subject is smaller than an average breathing signal of the subject.

The sleep breathing pattern information generation unit 222 may detect the section in which the radar signal is smaller than the average breathing signal and generate breathing state information about sleep breathing of the subject. That is, the sleep breathing pattern information generation unit 222 may generate sleep breathing state information of the subject that compares current sleep breathing of the subject with usual sleep breathing of the subject by using a radar. For example, the sleep breathing pattern information generation unit 222 may use Equation 2 below.

<Equation 2>

$$a_1[i] = \begin{cases} 1 & when \ x[i] < m[i] \\ 0 & otherwise \end{cases}$$

In Equation 2, $a_1$ is breathing state information about sleep breathing of a subject, x[i] is the radar signal reflected and received from the subject, and m[j] is an average breathing signal of the subject.

For example, the sleep breathing pattern information generation unit 222 may compare the radar signal received from the subject during sleep with the average breathing signal of the subject. The sleep breathing pattern information generation unit 222 may detect a section in which the radar signal received from the subject is smaller than the average breathing signal of the subject.

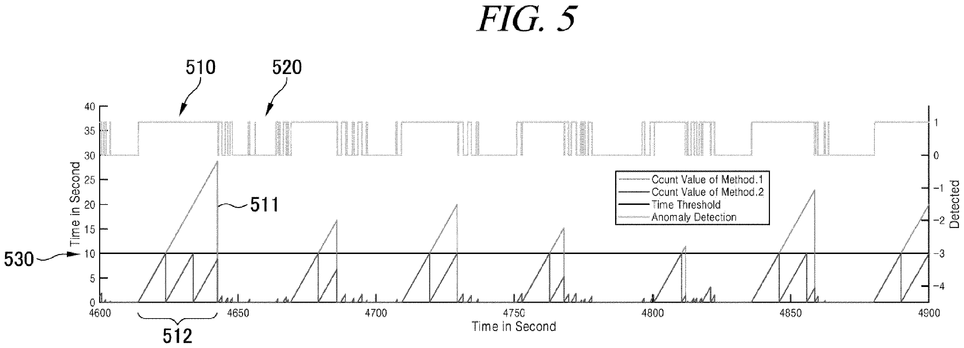

The sleep breathing pattern information generation unit 222 may generate first sleep breathing pattern information and second sleep breathing pattern information that represent the continuity of sleep state of the subject by using the breathing state information about sleep breathing of the subject. Hereafter, a process of generating sleep breathing pattern information of a subject will be described with reference to **FIG. 5.**

**FIG. 5** is a diagram for explaining first sleep breathing pattern information and second sleep breathing pattern information according to an embodiment of the present disclosure. Referring to **FIG. 5,** the sleep breathing pattern information generation unit 222 may first generate an average breathing signal of a subject. For example, if a radar signal is smaller than an average breathing signal, current breathing state information of the subject may be calculated as "1" (corresponding to reference numeral 510 in **FIG. 5**), and if the radar signal is greater than the average breathing signal, current breathing state information of the subject may be calculated as "0" (corresponding to reference numeral 512 in **FIG. 5**).

Then, the sleep breathing pattern information generation unit 222 may compare the radar signal reflected from the subject with the average breathing signal of the subject and generate sleep breathing pattern information including first sleep breathing pattern information 511 and second sleep breathing pattern information 512.

The sleep breathing pattern information generation unit 222 may generate the first sleep breathing pattern information 511 of the subject based on whether the breathing state information continues. The sleep breathing pattern information generation unit 222 may generate the first sleep breathing pattern information 511 having a value that is increased when current sleep breathing of the subject is lower than usual sleep breathing. For example, the sleep breathing pattern information generation unit 222 may generate the first sleep breathing pattern information 511 by using Equation 3 below.

<Equation 3>

$$c_{11}[i] = \begin{cases} c_{11}[i-1] + 1 & when \ a_1[i] = 1 \ and \ a_1[i-1] = 1 \\ 0 & otherwise \end{cases}$$

In Equation 3, $c_{11}$ is the first sleep breathing pattern information 511. For example, referring to Equation 3 and **FIG. 5,** the sleep breathing pattern information generation unit 222 may calculate the first sleep breathing pattern information 511 having a value that is increased when the radar signal reflected from the subject, i.e., current breathing of the subject, is smaller than the average breathing signal of the subject and is maintained. That is, the first sleep breathing pattern information 511 may have a value that is increased when current breathing state information of the subject is maintained at "1" (corresponding to reference numeral 510 in **FIG. 5**) and may refer to the entire section in which the value is smaller than the average breathing signal during sleep of the subject.

For another example, referring to Equation 3 and **FIG. 5,** the sleep breathing pattern information generation unit 222 may initialize the first sleep breathing pattern information 511 when current breathing of the subject is greater than the average breathing signal of the subject. That is, the first sleep breathing pattern information 511 may initialize the first sleep breathing pattern information 511 when the breathing state information of the subject is not "1" (corresponding to reference numeral 520 in **FIG. 5**).

The sleep breathing pattern information generation unit 222 may generate the second sleep breathing pattern information 512 based on whether the breathing state information continues and a predetermined threshold value. The sleep breathing pattern information generation unit 222 may generate the second sleep breathing pattern information 512 having a value that is increased when current sleep breathing of the subject is lower than usual sleep breathing, initialized at a specific threshold time and then increased again. For example, the sleep breathing pattern information generation unit 222 may generate the second sleep breathing pattern information 512 by using Equation 4 below.

<Equation 4>

$$c_{12}[i] = \begin{cases} c_{12}[i-1]+1 & when\ a_1[i]=1\ and\ a_1[i-1]=1 \\ & and\ c_{12}[i-1] < T \\ 0 & otherwise \end{cases}$$

In Equation 4, $c_{12}$ is the second sleep breathing pattern information 512 and T is a specific threshold time 530 as a predetermined threshold value. For example, referring to Equation 4 and **FIG. 5,** the sleep breathing pattern information generation unit 222 may calculate the second sleep breathing pattern information 512 having a value that is increased when the radar signal reflected from the subject, i.e., current breathing of the subject, is smaller than the average breathing signal of the subject and is maintained like the first sleep breathing pattern information 511, but is initialized at the specific threshold time 530 (T in Equation 4) and then increased again.

For example, referring to Equation 4 and **FIG. 5,** the sleep breathing pattern information generation unit 222 may increase the value of the second sleep breathing pattern information 512 when current breathing state information of the subject is maintained at "1" 510, and may initialize the value of the second sleep breathing pattern information 512 at the specific threshold time 530 and then increase the value again.

Also, the sleep breathing pattern information generation unit 222 may detect a case where a radar signal is greater than an average breathing signal and generate motion state information about sleep breathing of the subject. For example, the sleep breathing pattern information generation unit 222 may generate first sleep motion pattern information and second sleep motion pattern information that represent the continuity of sleep motion state of the subject by using the motion state information about a motion detected during sleep of the subject.

First, the sleep breathing pattern information generation unit 222 may detect a case where the radar signal is greater than the average breathing signal and generate motion state information about sleep breathing of the subject. That is, the sleep breathing pattern information generation unit 222 may generate sleep motion state information of the subject that compares current sleep breathing of the subject with usual sleep breathing of the subject by using a radar. For example, the sleep breathing pattern information generation unit 222 may use Equation 5 below.

<Equation 5>

$$a_2[i] = \begin{cases} 1 & when\ x[i] > m[i] \\ 0 & otherwise \end{cases}$$

In Equation 5, $a_2$ is motion state information about sleep breathing of a subject, x[i] is the radar signal reflected and received from the subject, and m[j] is an average breathing signal of the subject.

For example, referring to Equation 5, the sleep breathing pattern information generation unit 222 may detect a section in which the radar signal received from the subject during sleep is greater than the average breathing signal of the subject and generate motion state information about sleep breathing of the subject, first sleep motion pattern information and second sleep motion pattern information based on the detection result.

The sleep breathing pattern information generation unit 222 may generate the first sleep motion pattern information about a sleep motion of the subject based on whether the motion state information continues. The sleep breathing pattern information generation unit 222 may generate the first sleep motion pattern information having a value that is increased when current sleep breathing of the subject is higher than usual sleep breathing. For example, the sleep breathing pattern information generation unit 222 may use Equation 6 below.

<Equation 6>

$$c_{21}[i] = \begin{cases} c_{21}[i-1]+1 & when\ a_2[i]=1\ and\ a_2[i-1]=1 \\ 0 & otherwise \end{cases}$$

In Equation 6, $c_{21}$ is first sleep motion pattern information and $a_2$ is motion state information about sleep breathing of a subject. For example, referring to Equation 6, the sleep breathing pattern information generation unit 222 may calculate the first sleep motion pattern information having a value that is increased when the radar signal reflected from the subject is greater than the average breathing signal of the subject and is maintained. That is, the first sleep motion pattern information may refer to the entire section in which the value is greater than the average breathing signal during sleep of the subject.

The sleep breathing pattern information generation unit 222 may generate second sleep motion pattern information based on whether the motion state information continues and a predetermined threshold value. The sleep breathing pattern information generation unit 222 may generate the second sleep motion pattern information having a value that is increased when current sleep breathing of the subject is higher than usual sleep breathing, initialized at a specific threshold time and then increased again. For example, the sleep breathing pattern information generation unit 222 may use Equation 7 below.

<Equation 7>
$$c_{22}[i] = \begin{cases} c_{22}[i-1] + 1 & when\ a_2[i] = 1\ and\ a_2[i-1] = 1 \\ & and\ c_{22}[i-1] < T \\ 0 & otherwise \end{cases}$$

In Equation 7, $c_{22}$ is second sleep motion pattern information and T is a specific threshold time as a predetermined threshold value. For example, referring to Equation 7, the sleep breathing pattern information generation unit 222 may calculate the second sleep motion pattern information having a value that is increased when the radar signal reflected from the subject is greater than the average breathing signal of the subject and is maintained like the first sleep motion pattern information, but is initialized at the specific threshold time and then increased again.

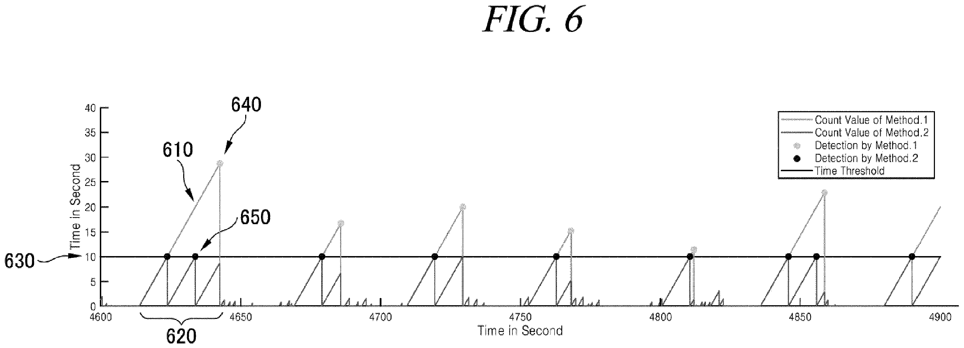

The sleep breathing event detection unit 223 may detect a sleep breathing event based on the sleep breathing pattern information. For example, the sleep breathing event detection unit 223 may detect a first sleep breathing event and a second sleep breathing event during sleep of the subject by using the first sleep breathing pattern information and the second sleep breathing pattern information. Hereafter, a process of generating sleep breathing pattern information of a subject will be described with reference to **FIG. 6.**

**FIG. 6** is a diagram for explaining a first sleep breathing event and a second sleep breathing event according to an embodiment of the present disclosure. Referring to **FIG. 6,** the sleep breathing analysis device 100 may detect a first sleep breathing event and a second sleep breathing event by detecting peak values 640 and 650 from first sleep breathing pattern information 610 and second sleep breathing pattern information 620.

The sleep breathing event detection unit 223 may detect the first sleep breathing event based on a value corresponding to a threshold value or more in the first sleep breathing pattern information 610. The sleep breathing event detection unit 223 may detect, as the first sleep breathing event, the number of breathings when sleep breathing of the subject is lower than usual in a period longer than a specific threshold time during sleep of the subject. For example, the sleep breathing event detection unit 223 may detect the first sleep breathing event by using Equation 8 below.

<Equation 8>
$$d_{11}[i] = \begin{cases} 1 & when\ c_{11}[i-1] \geq T\ and\ c_{11}[i-1] > c_{11}[i] \\ 0 & otherwise \end{cases}$$

In Equation 8, $d_{11}$ is a first sleep breathing event, $c_{11}$ is the first sleep breathing pattern information 610, and T is a specific threshold time 630 as a predetermined threshold value. Referring to Equation 8 and **FIG. 6,** the sleep breathing analysis device 100 may detect and count the peak value 640 from the first sleep breathing pattern information 610 in a period longer than the specific threshold time 630 and thus detect the number of first sleep breathing events.

The sleep breathing event detection unit 223 may detect the second sleep breathing event based on a value reaching the threshold value in the second sleep breathing pattern information 620. The sleep breathing event detection unit 223 may detect, as the second sleep breathing event, the number of sleep breathings reaching the specific threshold time during sleep of the subject. For example, the sleep breathing analysis device 100 may detect the second sleep breathing event by using Equation 9 below.

<Equation 9>
$$d_{12}[i] = \begin{cases} 1 & when\ c_{12}[i] = T \\ 0 & otherwise \end{cases}$$

In Equation 9, $d_{12}$ is a second sleep breathing event, $c_{12}$ is the second sleep breathing pattern information 620, and T is the specific threshold time 630 as a predetermined threshold value. Referring to Equation 9 and **FIG. 6,** the sleep breathing analysis device 100 may detect and count the peak value 650, which reaches the specific threshold time 630, from the second sleep breathing pattern information 620 and thus detect the number of second sleep breathing events.

That is, the sleep breathing event detection unit 223 may express a pattern depending on the duration of an apnea/hypopnea event by detecting the first sleep breathing event and the second sleep breathing event in a mixed manner.

Also, the sleep breathing event detection unit 223 may detect a sleep motion event based on sleep motion pattern information. The sleep breathing event detection unit 223 may detect a first sleep motion event based on a value corresponding to a threshold value or more in the first sleep motion pattern information. The sleep breathing event detection unit 223 may detect, as the first sleep motion event, the number of motions when sleep breathing of the subject is higher than usual in a period longer than a specific threshold time during sleep of the subject. For example, the sleep breathing event detection unit 223 may use Equation 10 below.

<Equation 10>

$$d_{21}[i] = \begin{cases} 1 & when\ c_{21}[i-1] \geq T\ and\ c_{21}[i-1] > c_{21}[i] \\ 0 & otherwise \end{cases}$$

In Equation 10, $d_{21}$ is a first sleep motion event, $c_{21}$ is a first sleep motion pattern information, and T is a specific threshold time as a predetermined threshold value. Referring to Equation 10, the sleep breathing event detection unit 223 may detect and count a peak value from the first sleep motion pattern information in a period longer than the specific threshold time and thus detect the number of first sleep motion events.

The sleep breathing event detection unit 223 may detect a second sleep motion event based on a value reaching the threshold value in the second sleep motion pattern information. The sleep breathing event detection unit 223 may detect, as the second sleep motion event, the number of sleep motions reaching the specific threshold time during sleep of the subject. For example, the sleep breathing event detection unit 223 may detect the second sleep motion event by using Equation 11 below.

<Equation 11>

$$d_{22}[i] = \begin{cases} 1 & when\ c_{22}[i] = T \\ 0 & otherwise \end{cases}$$

In Equation 11, $d_{22}$ is a second sleep motion event, $c_{22}$ is a second sleep motion pattern information, and T is a specific threshold time as a predetermined threshold value. Referring to Equation 11, the sleep breathing event detection unit 223 may detect and count a peak value, which reaches the specific threshold time, from the second sleep motion pattern information and thus detect the number of second sleep motion events.

That is, the sleep breathing event detection unit 223 may suppress a detection error of a sleep breathing event by detecting the first sleep motion event and the second sleep motion event in a mixed manner.

The sleep breathing event detection unit 223 may set optimal parameters for a plurality of sleep items (or sleep test items). For example, the sleep breathing event detection unit 223 may set sleep test items that can be extracted by using a radar. The sleep breathing event detection unit 223 may set various sleep test items including apnea, hypopnea, central sleep apnea that causes apnea and hypopnea, and various temporary arousal and sleep stages based on human abdominal and limb motions.

The optimal parameters may include event occurrence-related parameters and signal processing parameters. For example, the event occurrence-related parameters may be regression coefficients for the first sleep breathing pattern information, the second sleep breathing pattern information, the first sleep motion pattern information and the second sleep motion pattern information detected during sleep of the subject, and the signal processing parameters correspond to, for example, F, T and L of Equation 1 and may be parameters for detecting each of the plurality of sleep items.

The sleep breathing event detection unit 223 may derive event occurrence information for the plurality of sleep items based on the optimal parameters. For example, the sleep breathing event detection unit 223 may calculate the number of events for each of the plurality of sleep items by varying the signal processing parameters.

For example, the sleep breathing event detection unit 223 may derive event occurrence information for the plurality of sleep items by using Equation 12 below.

<Equation 12>

$$Y = b_0 + b_1 N_{11} + b_2 N_{12} + b_3 N_{21} + b_4 N_{22} + e$$

For example, in Equation 12, Y may be the total number of events corresponding to event occurrence information, and $b_0$ to $b_4$ may be calculated by using a least squares method as event occurrence-related parameters. $N_{11}$ to $N_{22}$ are the numbers of events respectively corresponding to a first sleep breathing event, a second sleep breathing event, a first sleep motion event and a second sleep motion event detected by the above-described methods, and e may be an error term.

For example, the sleep breathing event detection unit 223 may detect a first sleep breathing event, a second sleep breathing event, a first sleep motion event and a second sleep motion event for an Central item based on the signal processing parameters F, L and T previously set for the Central item related to apnea, and may derive event occurrence information for the Central item related to apnea by substituting the preset event occurrence-related parameters and the total number of first sleep breathing events, the total number of second sleep breathing events, the total number of first sleep motion events, and the total number of second sleep motion events detected for the Central item into Equation 12.

The index calculation unit 230 may calculate breathing-related index information based on sleep time information and the event occurrence information for the plurality of sleep items. For example, the sleep time information may correspond to the entire sleep time of a test subject.

The index calculation unit 230 may calculate the breathing-related index information by dividing the total number of event occurrence information calculated for the plurality of sleep items by the sleep time information.

The index calculation unit 230 may calculate breathing-related index information including apnea-hypopnea index information and RDI information. The index calculation unit 230 may calculate the apnea-hypopnea index information based on event occurrence information for an apnea item among the plurality of sleep items and event occurrence information related to a hypopnea item. For example, the index calculation unit 230 may use Equation 13 below.

<Equation 13>

$$AHI = \frac{\sum Number\ of\ apneas\ + \sum Number\ of\ hypopneas}{Entire\ sleep\ time}$$

In Equation 13, *AHI* is apnea-hypopnea index (AHI) information, $\Sigma$*Number of apneas* is the total sum of event occurrence information for detection items detected related to apnea, and $\Sigma$ *Number of hypopneas* is the total sum of event occurrence information for detection items detected related to hypopnea.

For example, referring to Equation 13, the index calculation unit 230 may calculate the number of apneas by summing up event occurrence information for a Central item, event occurrence information for a Mixed item and event occurrence information for an Obstructive item detected related to apnea which are detected related to apnea, and may calculate the number of hypopneas by summing up event occurrence information for a Central item, event occurrence information for a Mixed item and event occurrence information for an Obstructive item which are detected related to hypopnea.

For example, the index calculation unit 230 may predict a sleep time of a subject by motion analysis based on a radar signal received from the subject. The index calculation unit 230 may calculate the apnea-hypopnea index information by dividing the number of apneas and the number of hypopneas by the entire sleep time of the subject.

The index calculation unit 230 may calculate the RDI information based on the event occurrence information for the apnea item among the plurality of sleep items, the event occurrence information for the Hypopnea item and event occurrence information for a Respiratory Effort item Respiratory Effort. For example, the index calculation unit 230 may use Equation 14 below.

<Equation 14>

$$RDI = \frac{\sum Number\ of\ apneas + \sum number\ of\ hypopneas + \sum Respiratory\ Effort\ Related\ Arousal}{Entire\ sleep\ time}$$

In Equation 14, *RDI* is respiratory disturbance index (RDI) information, and $\Sigma$ *Number of apneas* , $\Sigma$ *number of hypopneas* and $\Sigma$ *Respiratory Effort Related Arousal* may be the total sums of event occurrence information for detection items detected related to apnea, hypopnea and respiratory effort related arousal, respectively.

For example, referring to Equation 14, the index calculation unit 230 may calculate the RDI information by dividing the number of apneas, the number of hypopneas and the number of respiratory effort related arousals detected from a radar signal received from the subject by the entire sleep time of the subject.

The disease determination unit 240 may determine whether the subject has a sleep-related disease based on the breathing-related index information. The disease determination unit 240 may compare a disease group distribution information of sleep-related disease and a control group distribution information with the breathing-related index information to determine whether the subject has a sleep-related disease. For example, the disease determination unit 240 may compare the disease group distribution information of sleep-related disease and the control group distribution information with the breathing-related index information by using a deep learning model. In this case, a disease group and a control group of the present disclosure may include a disease group with Alzheimer's dementia and a control group.

For example, the disease determination unit 240 may use distribution values of sleep components of the disease group with sleep-related disease and distribution values of sleep components of the control group. For example, the disease determination unit 240 may use the mean and standard deviation as the distribution values of sleep components of the disease group and control group. The disease determination unit 240 may compare the apnea-hypopnea index information with the mean and standard deviation of the disease group with related diseases and compare the apnea-hypopnea index information with the mean and standard deviation of the control group.

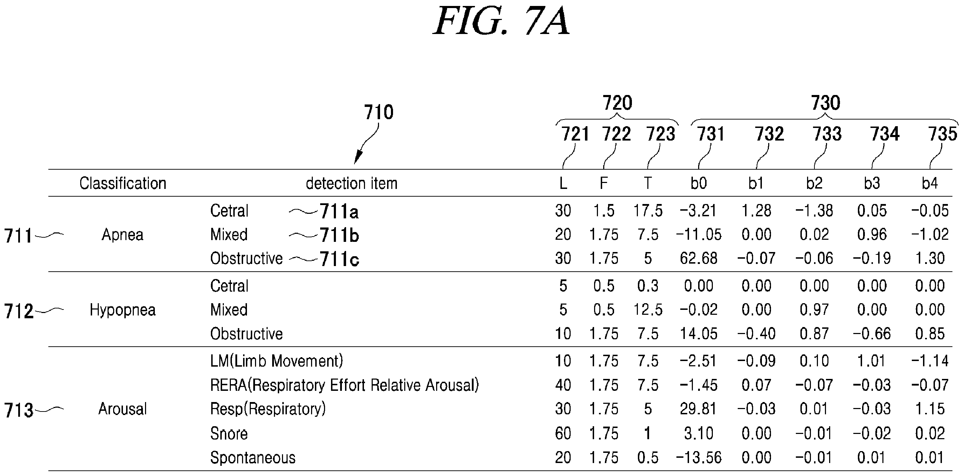

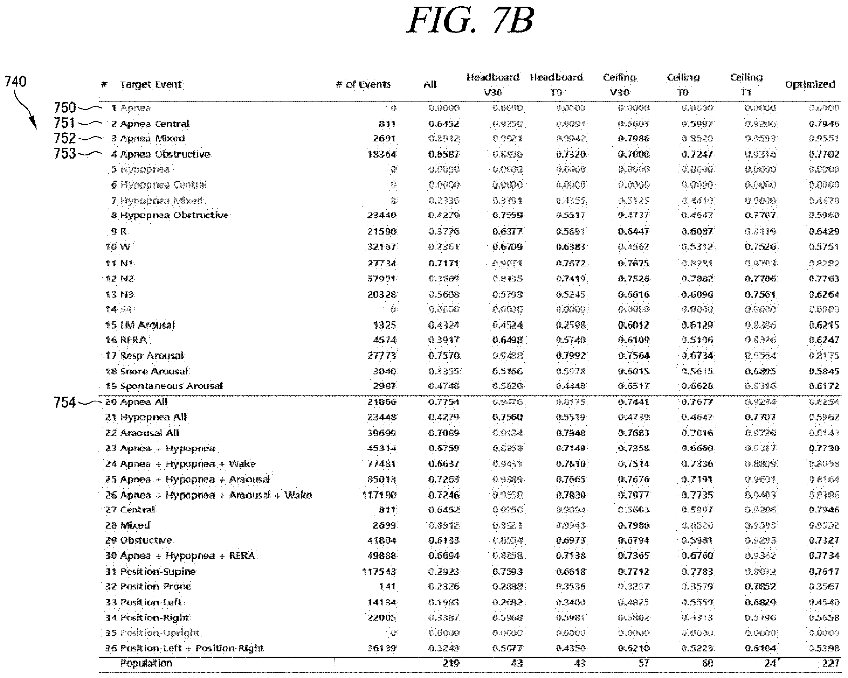

For example, the disease determination unit 240 may determine whether the subject has sleep-related disease based on the comparison result. The disease determination unit 240 may compare the apnea-hypopnea index information of the subject with distribution information of a disease group with apnea or distribution information of a control group and determine whether the subject has sleep apnea based on the comparison result. **FIG.** 7A is a diagram for explaining optimal parameters for a plurality of sleep items according to an embodiment of the present disclosure, and **FIG. 7B** is a diagram showing an example of a data set for polysomnography according to an embodiment of the present disclosure.

Referring to **FIG. 7A** and **FIG. 7B**, the sleep breathing analysis device 100 may set a plurality of sleep items 710 that can be detected by using a radar and set optimal parameters for the respective items. The sleep breathing analysis device 100 may derive event occurrence information based on the optimal parameters set for the plurality of sleep items 710.

First, as shown in **FIG. 7A,** the sleep breathing analysis device 100 may set detection items 710 for each of a plurality of sleep items related to Apnea 711, Hypopnea 712 and Arousal 713. The sleep breathing analysis device 100 may set an Central item 711a, an Mixed item 711b and an Obstructive item 711c, which cause Apnea 711, as the detection items 710 for Apnea 711, set an Central item, an Mixed item and an Obstructive item, which cause Hypopnea 712, as the detection items 710 for Hypopnea 712, and set a Limb Movement (LM) item, a Respiratory Effort Related Arousal (RERA) item, a Respiratory (Resp) item, a Snore item and a Spontaneous time, which cause Arousal 713, as the detection items 710 for Arousal 713.

For example, the sleep breathing analysis device 100 may set optimal parameters including signal processing parameters 720 and event occurrence-related parameters 730 for the detection items 710 set for each of the plurality of sleep items.

As shown in **FIG. 7A,** the sleep breathing analysis device 100 may set the signal processing parameters 720 including a time sensitivity factor that means the length of time between specific consecutive periods, L 721, an amplitude sensitivity factor that responds to the amplitude between the specific consecutive periods, F 722, a specific threshold time and T 723.

The sleep breathing analysis device 100 may set the event occurrence-related parameters 730 including a regression coefficient $b_1$ 732 for a first sleep breathing event, a regression coefficient $b_2$ 733 for a second sleep breathing event, a regression coefficient $b_3$ 734 for a first sleep motion event, a regression coefficient $b_4$ 735 for a second sleep motion event, other regression coefficients $b_0$ 731 which area detected from the radar signal reflected from the subject.

For example, the sleep breathing analysis device 100 may calculate event occurrence information by varying the signal processing parameters 720 for the plurality of sleep items and detect an optimal factor and a regression coefficient for each sleep item by regression analysis. For example, L 721 may be one of 10s, 20s, 30s, 40s, 50s and 60s, F 722 may be one of 0.5, 0.75, 1, 1.25, 1.5, 1.75 and 2, and T 723 may be one of 5s, 7.5s, 10s, 12.5s, 15s, 17.5s and 20s to vary the signal processing parameters 720.

Referring to **FIG. 7B,** the sleep breathing analysis device 100 may set optimal parameters for the plurality of sleep items based on a data set 740 for polysomnography.

For example, the sleep breathing analysis device 100 may set optimal parameters including the signal processing parameters 720 and the event occurrence-related parameters 730 for the Central item 711a related to Apnea 711 based on the data set 740 shown in **FIG. 7B.**

For example, referring to **FIG. 7A** and **FIG. 7B,** the sleep breathing analysis device 100 may set the signal processing parameters 720 and the event occurrence-related parameters 730 for the Central item 711a related to Apnea 711 based on information about an apnea Central item 751 that causes Apnea 750 measured from the data set 740 by polysomnography, and may set the signal processing parameters 720 and the event occurrence-related parameters 730 for the Mixed item 711b related to Apnea 711 based on information about an apnea Mixed item 752 that causes Apnea 750 measured by polysomnography.

Further, the sleep breathing analysis device 100 may set the signal processing parameters 720 and the event occurrence-related parameters 730 for Apnea 711 based on information about Apnea All 754 measured by polysomnography.

For example, referring to **FIG. 7A** and **FIG. 7B,** the sleep breathing analysis device 100 may determine a factor with the highest correlation coefficient with a correct answer value measured by polysomnography as an optimal factor and thus may set the signal processing parameters 720 including the detection items 710 for Apnea 711, the time sensitivity factor, L 721 of "30s", the amplitude sensitivity factor, F 722 of "1.5", the specific threshold time and T 723 of "17.5" for the Central item 711a as optimal parameters with reference to the data set 740, and may set the event occurrence-related parameters 730 including the regression coefficients, $b_0$ 731 of "-3.21", $b_1$ 732 of "1.28", $b_2$ 733 of "-1.38", $b_3$ 734 of "0.05" and $b_4$ 735 of "-0.05".

For example, the sleep breathing analysis device 100 may derive event occurrence information for the plurality of sleep items based on the optimal parameters set for the plurality of sleep items (see Equation 12 above). Specifically, the sleep breathing analysis device 100 may derive event occurrence information for the Central item related to apnea of the subject by using information detected related to the Central item 711a of Apnea 711, the first sleep breathing event, the second sleep breathing event, the first sleep motion event and the second sleep motion event, and the optimal parameters 720 and 730 set for the Central item 711a of Apnea 711.

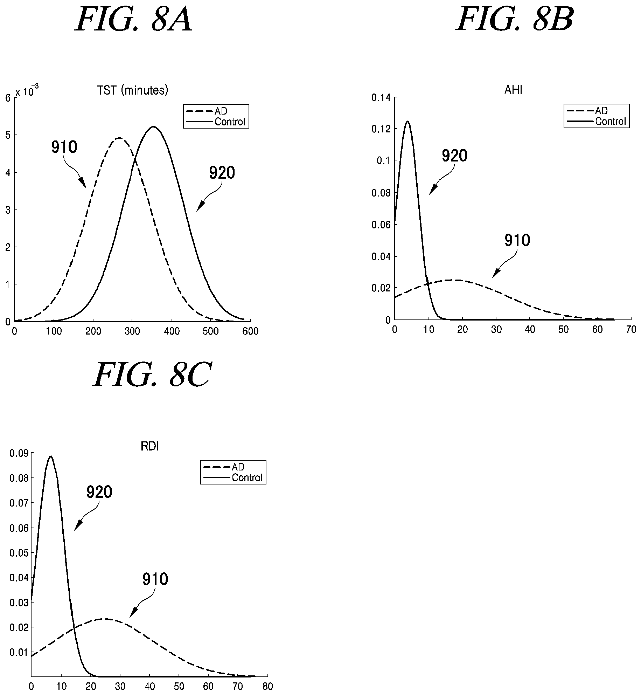

**FIG. 8** shows examples of distribution information of sleep-related disease of according to an embodiment of the present disclosure. **FIG. 8A** shows distribution information of total sleep time TST of a disease group with Alzheimer's dementia and a control group, **FIG. 8B** shows distribution information of apnea-hypopnea of the disease group with Alzheimer's dementia and the control group, and **FIG. 8C** shows distribution information of respiratory disturbance of the disease group and the control group with Alzheimer's dementia. The distribution information shown in **FIG. 8A** to **FIG. 8C** may be graphed by processing data about the total sleep time, apnea-hypopnea index and RDI of Alzheimer's dementia, and may include mean and standard deviation.

For example, the sleep breathing analysis device 100 may compare the distribution information of sleep-related disease and determine whether the subject is included in the disease group with the corresponding sleep-related disease.

For example, referring to **FIG. 8A,** the sleep breathing analysis device 100 may compare the total sleep time of the subject with distribution information 810 of the total sleep time of the disease group with Alzheimer's dementia, and may compare the total sleep time of the subject with distribution information 820 of the total sleep time of the control group. The sleep breathing analysis device 100 may determine whether distribution information of the total sleep time of the subject is included in the disease group with Alzheimer's dementia based on the comparison result.

For example, referring to **FIG. 8B,** the sleep breathing analysis device 100 may compare the apnea-hypopnea index information detected from the subject with distribution information 830 of apnea-hypopnea of the disease group with Alzheimer's dementia, and may compare the apnea-hypopnea index information of the subject with distribution information 840 of apnea-hypopnea of the control group. The sleep breathing analysis device 100 may determine whether the apnea-hypopnea index information of the subject is included in the disease group with Alzheimer's dementia based on the comparison result.

For example, referring to **FIG. 8C,** the sleep breathing analysis device 100 may compare RDI information detected from the subject with distribution information 850 of respiratory disturbance of the disease group with Alzheimer's dementia, and may compare the RDI information of the subject with distribution information 860 of respiratory disturbance of the control group. The sleep breathing analysis device 100 may determine whether the RDI information of the subject is included in the disease group with Alzheimer's dementia based on the comparison result.

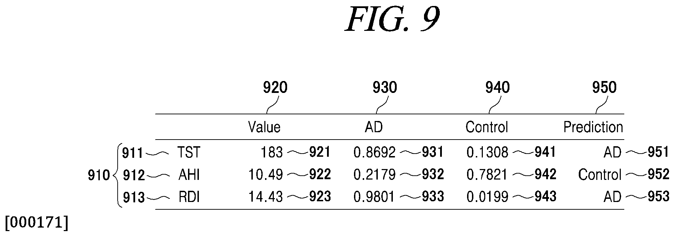

**FIG. 9** is a diagram for explaining a process of determining whether a subject has sleep-related disease according to an embodiment of the present disclosure. Referring to **FIG. 9**, the sleep breathing analysis device 100 may compare breathing-related index information 920 detected from the subject for a plurality of breathing-related index items 910 with each of distribution information of a disease group with sleep-related disease and distribution information of a control group, and may generate prediction information 950 by determining whether the subject has sleep-related disease in terms of probability distribution based on the comparison result.

For example, the sleep breathing analysis device 100 may compare the index information 920 of the subject for the plurality of breathing-related index items 910 with the distribution information of the disease group with sleep-related disease to detect information 930 about the probability of the subject being included in the disease group, and may compare the index information 920 of the subject for the plurality of breathing-related index items 910 with the distribution information of the control group to detect information 940 about the probability of the subject being included in a normal group. The sleep breathing analysis device 100 may generate the prediction information 950 by determining whether the subject is included in the disease group with sleep-related disease based on the detected probability information 930 and 940.

Referring to the example shown in **FIG. 9,** the sleep breathing analysis device 100 may calculate information "0.8692" 931 about the probability of being included in the disease group and information "0.1308" 941 about the probability of being included in the control group based on the result of comparing index information "183" 921 of total sleep time 911 of the subject with the distribution information of the total sleep time of the disease group and the distribution information of the total sleep time of the control group. Accordingly, the sleep breathing analysis device 100 may generate prediction information "AD" 951 by determining that the subject is included in the disease group with sleep-related disease in terms of probability distribution based on the total sleep time of the subject.

The sleep breathing analysis device 100 may calculate information "0.2179" 932 about the probability of being included in the disease group and information "0.7821" 942 about the probability of being included in the control group based on the result of comparing index information "10.49" 922 of an apnea-hypopnea index 912 of the subject with the distribution information of apnea-hypopnea of the disease group and the distribution information of apnea-hypopnea of the control group. Accordingly, the sleep breathing analysis device 100 may generate prediction information "Control" 952 by determining that the subject is included in the normal group in terms of probability distribution based on the apnea-hypopnea index information of the subject.

The sleep breathing analysis device 100 may calculate information "0.9801" 933 about the probability of being included in the disease group and information "0.0199" 943 about the probability of being included in the control group based on the result of comparing index information "14.43" 923 of RDI 913 of the subject with the distribution information of respiratory disturbance of the disease group and the distribution information of respiratory disturbance of the control group. Accordingly, the sleep breathing analysis device 100 may generate prediction information "AD" 953 by determining that the subject is included in the disease group in terms of probability distribution based on the RDI information of the subject.

In this case, a final determination result as to the disease group/control group for each item may be determined by voting. For example, the total sleep time 911 and the RDI 913 of the subject are determined as being included in the disease group and the apnea-hypopnea index 912 is determined as being included in the normal group, and, thus, the subject is predominantly determined as being included in the disease group for each item. Therefore, the subject is finally determined as being included in the disease group and diseases are determined.

As described above, according to the present disclosure, it is possible to determine a disease by using the result of analyzing a radar signal during sleep of a subject, and also possible to provide a disease determination service for providing the subject with information about the degermation of disease.

Besides, information that becomes a basis for determination may be gathered after the degermation of disease. The gathered information may also provide a basis for determination of disease to the user at the time of service. The location of the subject and the probability may be expressed on a specific domain information by refining the items simply used for test and criteria for determination of disease for each item.

Also, more detailed information may be derived and may be provided to the subject. The testee (subject) is included in the normal group in terms of the apnea-hypopnea index (AHI) 912, but is determined as being included in the disease group with a very high probability in terms of the RDI 913 to which the average number of RERAs is added. That is, the number of RERAs contributes greatly to an increase in the RDI, and the RERA is very related to central sleep apnea. Therefore, the results of items related to "Central item" among a plurality of sleep test items may be provided together to the subject.

When data on various cases are accumulated later in addition to the above-described information, statistical information such as disease location in a similar age group, a similar home environment group and a similar underlying disease group may be provided together.

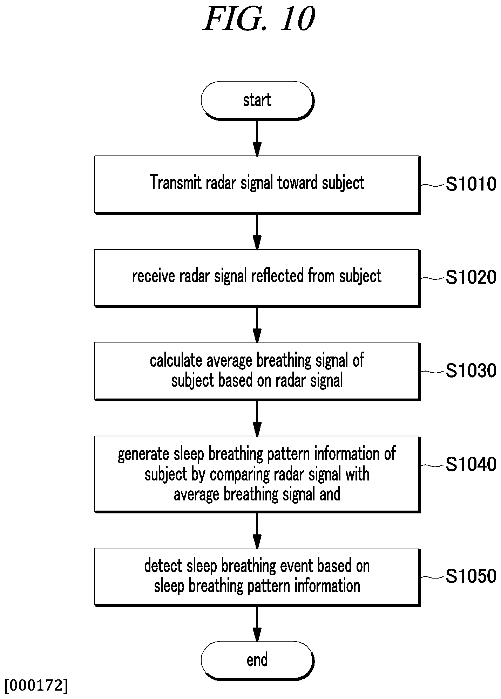

**FIG. 10** is a flowchart showing a sleep breathing analysis method according to an embodiment of the present disclosure. The method for analyzing sleep breathing using a radar illustrated in **FIG. 10** includes the processes time-sequentially performed according to the embodiment illustrated **in FIG. 1** to **FIG. 9.** Therefore, the above descriptions of the processes may also be applied to the method for analyzing sleep breathing by the sleep breathing analysis device according to the embodiment illustrated in **FIG. 1** to **FIG. 9,** even though they are omitted hereinafter.

In a process S1010, the sleep breathing analysis device may transmit a radar signal toward a subject.

In a process S1020, the sleep breathing analysis device may receive the radar signal reflected from the subject.

In a process S1030, the sleep breathing analysis device may calculate an average breathing signal of the subject based on the radar signal.

In a process S1040, the sleep breathing analysis device may compare the radar signal with the average breathing signal and generate sleep breathing pattern information of the subject.

In a process S1050, the sleep breathing analysis device may detect a sleep breathing event based on the sleep breathing pattern information.

In the descriptions above, the processes S1010 to S1050 may be divided into additional processes or combined into fewer processes depending on an embodiment. In addition, some of the processes may be omitted and the sequence of the processes may be changed if necessary.

The method for analyzing sleep breathing by the sleep breathing analysis device using a radar illustrated in **FIG. 1** to **FIG. 10** can be implemented as a computer program stored in a computer-readable storage medium to be executed by a computer or a storage medium including instructions executable by a computer. Also, the method for analyzing sleep breathing by the sleep breathing analysis device using a radar illustrated in **FIG. 1** to **FIG. 10** can be implemented as a computer program stored in a computer-readable storage medium to be executed by a computer.

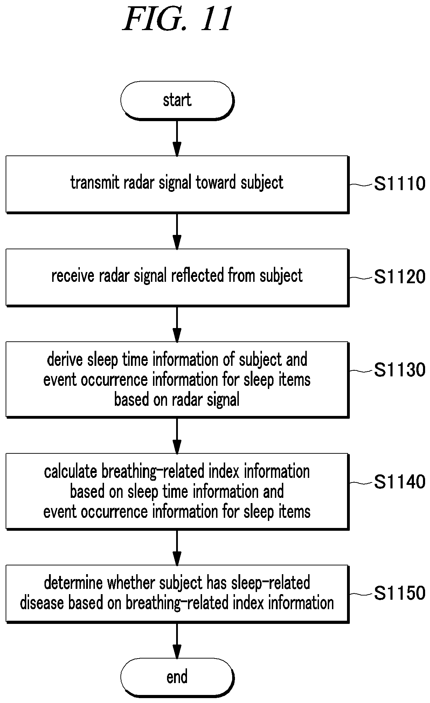

**FIG. 11** is a flowchart showing a disease determination method according to an embodiment of the present disclosure. The method for determination of disease using a radar illustrated in **FIG. 11** includes the processes time-sequentially performed according to the embodiment illustrated in **FIG. 1** to **FIG. 9.** Therefore, the above descriptions of the processes may also be applied to the method for determination of disease by the sleep breathing analysis device according to the embodiment illustrated in **FIG. 1** to **FIG. 9,** even though they are omitted hereinafter.

In a process S1110, the sleep breathing analysis device may transmit a radar signal toward a subject.

In a process S1120, the sleep breathing analysis device may receive the radar signal reflected from the subject.

In a process S1130, the sleep breathing analysis device may derive sleep time information of the subject and breathing-related index information for a plurality of sleep items based on the radar signal.

In a process S1140, the sleep breathing analysis device may calculate breathing-related index information based on the sleep time information and the event occurrence information for the plurality of sleep items.

In a process S1150, the sleep breathing analysis device may determine whether the subject has a sleep-related disease based on the breathing-related index information.

In the descriptions above, the processes S1110 to S1150 may be divided into additional processes or combined into fewer processes depending on an embodiment. In addition, some of the processes may be omitted and the sequence of the processes may be changed if necessary.

The method for determining disease using a radar in a sleep breath analysis device described above with reference to FIG. 1 to FIG. 11 can be implemented in a computer program stored in a medium to be executed by a computer or a storage medium including instructions codes executable by a computer. Also, the method for determining disease using a radar in a sleep breath analysis device described above with reference to FIG. 1 to FIG. 11 can be implemented in a computer program stored in a medium to be executed by a computer.

A computer-readable medium can be any usable medium which can be accessed by the computer and includes all volatile/non-volatile and removable/non-removable media. Further, the computer-readable medium may include all computer storage and communication media. The computer storage medium includes all volatile/non-volatile and removable/non-removable media embodied by a certain method or technology for storing information such as computer-readable instruction code, a data structure, a program module or other data. The communication medium typically includes the computer-readable instruction code, the data structure, the program module, or other data of a modulated data signal such as a carrier wave, or other transmission mechanism, and includes a certain information transmission medium.

The above description of the present disclosure is provided for the purpose of illustration, and it would be understood by those skilled in the art that various changes and modifications may be made without changing technical conception and essential features of the present disclosure. Thus, it is clear that the above-described embodiments are illustrative in all aspects and do not limit the present disclosure. For example, each component described to be of a single type can be implemented in a distributed manner. Likewise, components described to be distributed can be implemented in a combined manner.

The scope of the present invention is defined by the following claims.

## Claims

1. A device (100) for analyzing sleep breathing using a radar (110), comprising:

   a) a transceiver (210) configured to transmit (S1010) a radar signal (310, 410) toward a subject (111) and receive the radar signal (310, 410) reflected from the subject (111);
   b) an average breathing signal calculation unit (221) configured to calculate an average breathing signal (311) of the subject (111) based on the reflected radar signal (310, 410);
   c) a sleep breathing pattern information generation unit (222) configured to generate sleep breathing pattern information (511, 512, 610, 620) of the subject (111) by comparing the radar signal (310, 410) with the average breathing signal (311); and
   d) a sleep breathing event detection unit (223) configured to detect a sleep breathing event based on the sleep breathing pattern information (511, 512, 610, 620);

   **characterized in that**:

   e) the sleep breathing pattern information generation unit (222) is further configured to generate breathing state information (510; 520) about sleep breathing of the subject (111) including:

      e1) generating a first sleep breathing pattern information (511) about sleep breathing of the subject (111) based on whether the breathing state information (510) continues; and
      e2) generating a second sleep breathing pattern information (512) based on whether the breathing state information continues (510) and a predetermined threshold value ,

   f) wherein the sleep breathing pattern information generation unit (222) is adapted to calculate the first sleep breathing pattern information (511) and the second sleep breathing pattern information (512) to have a value that is continued to be increased over time while the radar signal (310, 410) is smaller than the average breathing signal (311), and

g) wherein the second sleep breathing pattern information (512) is initialized at the predetermined threshold value and then increased again, and

h) wherein the predetermined threshold value is a specific threshold time.

2. The device (100) of Claim 1,
   wherein the average breathing signal (311) calculation unit is configured to calculate the average breathing signal (311) of the subject (111) based on a time sensitivity factor (412, 413) and an amplitude sensitivity factor (415, 416) of the radar signal (310, 410).

3. The device (100) of Claim 1,
   wherein the sleep breathing event detection unit (223) is further configured to detect:

   a first sleep breathing event based on a value (640, 650) corresponding to the threshold value or more in the first sleep breathing pattern information (511); and
   a second sleep breathing event based on a value (640, 650) reaching the threshold value in the second sleep breathing pattern information (512).

4. The device (100) of any one of Claims 1 to 3,
   wherein the sleep breathing pattern information generation unit (222) is further configured to generate motion state information about sleep breathing of the subject (111) by detecting a case where the radar signal (310, 410) is greater than the average breathing signal (311).

5. The device (100) of Claim 4,
   wherein the sleep breathing pattern information generation unit (222) is further configured to generate:

   first sleep motion pattern information about a sleep motion of the subject (111) based on whether the motion state information continues; and
   second sleep motion pattern information based on whether the motion state information continues and a predetermined threshold value being a specific threshold time.

6. The device (100) of Claim 5,
   wherein the sleep breathing event detection unit (223) is further configured to detect:

   a first sleep motion event based on a value (640, 650) corresponding to the threshold value or more in the first sleep motion pattern information; and
   a second sleep motion event based on a value (640, 650) reaching the threshold value in the second sleep motion pattern information.

7. A method for analyzing sleep breathing using a radar (110), comprising:

   a) transmitting (S1010) a radar signal (310, 410) toward a subject (111);
   b) receiving (S1020) the radar signal (310, 410) reflected from the subject (111);
   c) calculating (S1030) an average breathing signal (311) of the subject (111) based on the reflected radar signal (310, 410) ;
   d) generating (S1040) sleep breathing pattern information (511, 512, 610, 620) of the subject (111) by comparing the radar signal (310, 410) with the average breathing signal (311); and
   e) detecting (S1050) a sleep breathing event based on the sleep breathing pattern information (511, 512, 610, 620);

   **characterized in that**:
   f) the generating sleep breathing pattern information (511, 512, 610, 620) includes generating sleep breathing state information (510, 520) about sleep breathing of the subject (111) including:

   g1) generating first sleep breathing pattern information (511) about sleep breathing of the subject (111) based on whether the breathing state information (510) continues;

g2) generating second sleep breathing pattern information (512) based on whether the breathing state information (312) continues and a predetermined threshold value; and

h) calculating the first sleep breathing pattern information (511) and the second sleep breathing pattern information (512) to have a value that is continued to be increased over time while the radar signal (310, 410) is smaller than the average breathing signal (311), and

i) wherein the second sleep breathing pattern information (512) is initialized at the predetermined threshold value and then increased again, and

h) wherein the predetermined threshold value is a specific threshold time.

8. The method for analyzing sleep breathing of Claim 7,
wherein in the calculating the average breathing signal (311), the average breathing signal (311) of the subject (111) is calculated based on a time sensitivity factor (412, 413) and an amplitude sensitivity factor (415, 416) of the radar signal (310, 410).

9. The method of Claim 7,
wherein the detecting a sleep breathing event further includes:

detecting a first sleep breathing event based on a value (640, 650) corresponding to the threshold value or more in the first sleep breathing pattern information (511, 512, 610, 620); and
detecting a second sleep breathing event based on a value (640, 650) reaching the threshold value in the second sleep breathing pattern information (511, 512, 610, 620).

10. The method of any one of Claims 7 to 9,
wherein the generating sleep breathing pattern information (511, 512, 610, 620) further includes generating motion state information about sleep breathing of the subject (111) by detecting a case where the radar signal (310, 410) is greater than the average breathing signal (311).

11. The method of Claim 10,
wherein the generating sleep breathing pattern information (511, 512, 610, 620) further includes:

generating first sleep motion pattern information about a sleep motion of the subject (111) based on whether the motion state information continues; and
generating second sleep motion pattern information based on whether the motion state information continues and a predetermined threshold value being a specific threshold time.

12. The method of Claim 11,
wherein the detecting a sleep breathing event further includes:

detecting a first sleep motion event based on a value (640, 650) corresponding to the threshold value or more in the first sleep motion pattern information; and
detecting a second sleep motion event based on a value (640, 650) reaching the threshold value in the second sleep motion pattern information.

13. A computer program stored in a computer-readable storage medium including a sequence of instructions for analyzing sleep breathing using a radar (110),
wherein the computer program includes a sequence of instructions that, when executed by a computing device, causes the device according to one or more of claims 1-6 to carry out the steps of the one or more of the method claims 7-12.

**Patentansprüche**

1. Vorrichtung (100) zum Analysieren von Schlafatmung unter Verwendung eines Radars (110), umfassend:

a) einen Senderempfänger (210), der konfiguriert ist, um ein Radarsignal (310, 410) zu einer Person (111) zu übertragen (S1010) und das von der Person (111) reflektierte Radarsignal (310, 410) zu empfangen;
b) eine Durchschnittsatmungssignalberechnungseinheit (221), die konfiguriert ist, um ein Durchschnittsatmungssignal (311) der Person (111) basierend auf dem reflektierten Radarsignal (310, 410) zu berechnen;

c) eine Schlafatmungsmusterinformationserzeugungseinheit (222), die konfiguriert ist, um Schlafatmungsmusterinformationen (511, 512, 610, 620) der Person (111) durch Vergleichen des Radarsignals (310, 410) mit dem Durchschnittsatmungssignal (311) zu erzeugen; und

d) eine Schlafatmungsereignisdetektionseinheit (223), die konfiguriert ist, um basierend auf den Schlafatmungsmusterinformationen (511, 512, 610, 620) ein Schlafatmungsereignis zu detektieren;

**dadurch gekennzeichnet, dass**

e) die Schlafatmungsmusterinformationserzeugungseinheit (222) ferner konfiguriert ist, um Atmungszustandsinformationen (510; 520) über die Schlafatmung der Person (111) zu erzeugen, einschließlich:

e1) Erzeugen erster Schlafatmungsmusterinformationen (511) über die Schlafatmung der Person (111) basierend darauf, ob die Atmungszustandsinformationen (510) fortdauern; und

e2) Erzeugen zweiter Schlafatmungsmusterinformationen (512) basierend darauf, ob die Atmungszustandsinformationen (510) fortdauern und auf einem vorbestimmten Schwellenwert,

f) wobei die Schlafatmungsmusterinformationserzeugungseinheit (222) angepasst ist, um die ersten Schlafatmungsmusterinformationen (511) und die zweiten Schlafatmungsmusterinformationen (512) zu berechnen, sodass sie einen Wert aufweisen, der im Laufe der Zeit fortwährend erhöht wird, während das Radarsignal (310, 410) kleiner als das Durchschnittsatmungssignal (311) ist, und

g) wobei die zweiten Schlafatmungsmusterinformationen (512) bei dem vorbestimmten Schwellenwert initialisiert und dann wieder erhöht werden, und

h) wobei der vorbestimmte Schwellenwert eine spezifische Schwellenzeit ist.

2. Vorrichtung (100) nach Anspruch 1,
wobei die Durchschnittsatmungssignalberechnungseinheit (311) konfiguriert ist, um das Durchschnittsatmungssignal (311) der Person (111) basierend auf einem Zeitempfindlichkeitsfaktor (412, 413) und einem Amplitudenempfindlichkeitsfaktor (415, 416) des Radarsignals (310, 410) zu berechnen.

3. Vorrichtung (100) nach Anspruch 1,
wobei die Schlafatmungsereignisdetektionseinheit (223) ferner konfiguriert ist, um Folgendes zu detektieren:

ein erstes Schlafatmungsereignis basierend auf einem Wert (640, 650) entsprechend dem Schwellenwert oder mehr in den ersten Schlafatmungsmusterinformationen (511); und
ein zweites Schlafatmungsereignis basierend auf einem Wert (640, 650), der den Schwellenwert in den zweiten Schlafatmungsmusterinformationen (512) erreicht.

4. Vorrichtung (100) nach einem der Ansprüche 1 bis 3,
wobei die Schlafatmungsmusterinformationserzeugungseinheit (222) ferner konfiguriert ist, um Bewegungszustandsinformationen über die Schlafatmung der Person (111) durch Detektieren eines Falls zu erzeugen, in dem das Radarsignal (310, 410) größer als das Durchschnittsatmungssignal (311) ist.

5. Vorrichtung (100) nach Anspruch 4,
wobei die Schlafatmungsmusterinformationserzeugungseinheit (222) ferner konfiguriert ist, um Folgendes zu erzeugen:

erste Schlafbewegungsmusterinformationen über eine Schlafbewegung der Person (111) basierend darauf, ob die Bewegungszustandsinformationen fortdauern; und
zweite Schlafbewegungsmusterinformationen basierend darauf, ob die Bewegungszustandsinformationen fortdauern, und einem vorbestimmten Schwellenwert, der eine spezifische Schwellenzeit ist.

6. Vorrichtung (100) nach Anspruch 5,
wobei die Schlafatmungsereignisdetektionseinheit (223) ferner konfiguriert ist, um Folgendes zu detektieren:

ein erstes Schlafbewegungsereignis basierend auf einem Wert (640, 650) entsprechend dem Schwellenwert oder mehr in den ersten Schlafbewegungsmusterinformationen; und
ein zweites Schlafbewegungsereignis basierend auf einem Wert (640, 650), der den Schwellenwert in den zweiten Schlafbewegungsmusterinformationen erreicht.

7. Verfahren zum Analysieren von Schlafatmung unter Verwendung eines Radars (110), umfassend:

a) Übertragen (S1010) eines Radarsignals (310, 410) zu einer Person (111);
b) Empfangen (S1020) des von der Person (111) reflektierten Radarsignals (310, 410);
c) Berechnen (S1030) eines Durchschnittsatmungssignals (311) der Person (111) basierend auf dem reflektierten Radarsignal (310, 410);
d) Erzeugen (S1040) von Schlafatmungsmusterinformationen (511, 512, 610, 620) der Person (111) durch Vergleichen des Radarsignals (310, 410) mit dem Durchschnittsatmungssignal (311); und
e) Detektieren (S1050) eines Schlafatmungsereignisses basierend auf den Schlafatmungsmusterinformationen (511, 512, 610, 620);

**dadurch gekennzeichnet, dass**
f) das Erzeugen von Schlafatmungsmusterinformationen (511, 512, 610, 620) ein Erzeugen von Schlafatmungszustandsinformationen (510, 520) über die Schlafatmung der Person (111) beinhaltet, einschließlich:

g1) Erzeugen erster Schlafatmungsmusterinformationen (511) über die Schlafatmung der Person (111) basierend darauf, ob die Atmungszustandsinformationen (510) fortdauern;
g2) Erzeugen zweiter Schlafatmungsmusterinformationen (512) basierend darauf, ob die Atmungszustandsinformationen (312) fortdauern und auf einem vorbestimmten Schwellenwert; und
h) Berechnen der ersten Schlafatmungsmusterinformationen (511) und der zweiten Schlafatmungsmusterinformationen (512), sodass sie einen Wert aufweisen, der im Laufe der Zeit fortwährend erhöht wird, während das Radarsignal (310, 410) kleiner als das Durchschnittsatmungssignal (311) ist, und
i) wobei die zweiten Schlafatmungsmusterinformationen (512) bei dem vorbestimmten Schwellenwert initialisiert und dann wieder erhöht werden, und
h) wobei der vorbestimmte Schwellenwert eine spezifische Schwellenzeit ist.

8. Verfahren zum Analysieren von Schlafatmung nach Anspruch 7,
wobei beim Berechnen des Durchschnittsatmungssignals (311) das Durchschnittsatmungssignal (311) der Person (111) basierend auf einem Zeitempfindlichkeitsfaktor (412, 413) und einem Amplitudenempfindlichkeitsfaktor (415, 416) des Radarsignals (310, 410) berechnet wird.

9. Verfahren nach Anspruch 7,
wobei das Detektieren eines Schlafatmungsereignisses ferner Folgendes beinhaltet:

Detektieren eines ersten Schlafatmungsereignisses basierend auf einem Wert (640, 650) entsprechend dem Schwellenwert oder mehr in den ersten Schlafatmungsmusterinformationen (511, 512, 610, 620); und
Detektieren eines zweiten Schlafatmungsereignisses basierend auf einem Wert (640, 650), der den Schwellenwert in den zweiten Schlafatmungsmusterinformationen (511, 512, 610, 620) erreicht.

10. Verfahren nach einem der Ansprüche 7 bis 9,
wobei das Erzeugen von Schlafatmungsmusterinformationen (511, 512, 610, 620) ferner ein Erzeugen von Bewegungszustandsinformationen über die Schlafatmung der Person (111) durch Detektieren eines Falls beinhaltet, in dem das Radarsignal (310, 410) größer als das Durchschnittsatmungssignal (311) ist.

11. Verfahren nach Anspruch 10,
wobei das Erzeugen von Schlafatmungsmusterinformationen (511, 512, 610, 620) ferner Folgendes beinhaltet:

Erzeugen erster Schlafbewegungsmusterinformationen über eine Schlafbewegung der Person (111) basierend darauf, ob die Bewegungszustandsinformationen fortdauern; und
Erzeugen zweiter Schlafbewegungsmusterinformationen basierend darauf, ob die Bewegungszustandsinformationen fortdauern, und einem vorbestimmten Schwellenwert, der eine spezifische Schwellenzeit ist.

12. Verfahren nach Anspruch 11,
wobei das Detektieren eines Schlafatmungsereignisses ferner Folgendes beinhaltet:

Detektieren eines ersten Schlafbewegungsereignisses basierend auf einem Wert (640, 650) entsprechend dem Schwellenwert oder mehr in den ersten Schlafbewegungsmusterinformationen; und
Detektieren eines zweiten Schlafbewegungsereignisses basierend auf einem Wert (640, 650), der den Schwellenwert in den zweiten Schlafbewegungsmusterinformationen erreicht.

**13.** Computerprogramm, das in einem computerlesbaren Speichermedium gespeichert ist, das eine Sequenz von Anweisungen zum Analysieren von Schlafatmung unter Verwendung eines Radars (110) beinhaltet,
wobei das Computerprogramm eine Sequenz von Anweisungen beinhaltet, die, wenn sie von einer Rechenvorrichtung ausgeführt werden, die Vorrichtung nach einem oder mehreren der Ansprüche 1-6 veranlassen, die Schritte des einen oder der mehreren der Verfahrensansprüche 7-12 auszuführen.

**Revendications**

**1.** Dispositif (100) pour analyser la respiration pendant le sommeil au moyen d'un radar (110), comprenant :

a) un émetteur-récepteur (210) prévu pour transmettre (S 1010) un signal radar (310, 410) vers un sujet (111) et recevoir le signal radar (310, 410) réfléchi par le sujet (111) ;
b) une unité de calcul de signal respiratoire moyen (221) prévue pour calculer un signal respiratoire moyen (311) du sujet (111) sur la base du signal radar réfléchi (310, 410) ;
c) une unité de génération d'informations de rythme respiratoire pendant le sommeil (222) prévue pour générer des informations de rythme respiratoire pendant le sommeil (511, 512, 610, 620) du sujet (111) par comparaison du signal radar (310, 410) avec le signal respiratoire moyen (311) ; et
d) une unité de détection d'événement respiratoire pendant le sommeil (223) prévue pour détecter un événement respiratoire pendant le sommeil sur la base des informations de rythme respiratoire pendant le sommeil (511, 512, 610, 620) ;

**caractérisé en ce que** :
e) l'unité de génération d'informations de rythme respiratoire pendant le sommeil (222) est prévue en outre pour générer des informations d'état respiratoire (510 ; 520) sur la respiration pendant le sommeil du sujet (111), comprenant :

e1) la génération d'une première information de rythme respiratoire pendant le sommeil (511) sur la respiration pendant le sommeil du sujet (111) sur la base de la continuation ou non des informations d'état respiratoire (510) ; et
e2) la génération d'une deuxième information de rythme respiratoire pendant le sommeil (512) sur la base de la continuation ou non des informations d'état respiratoire (510) et d'une valeur seuil prédéterminée ;
f) où l'unité de génération d'informations de rythme respiratoire pendant le sommeil (222) est adaptée pour calculer la première information de rythme respiratoire pendant le sommeil (511) et la deuxième information de rythme respiratoire pendant le sommeil (512) pour obtenir une valeur continuant à croître dans le temps pendant que le signal radar (310, 410) est inférieur au signal respiratoire moyen (311), et
g) où la deuxième information de rythme respiratoire pendant le sommeil (512) est initialisée à la valeur seuil prédéterminée avant de croître à nouveau, et
h) où la valeur seuil prédéterminée est un temps seuil spécifique.

**2.** Dispositif (100) selon la revendication 1,
où l'unité de calcul de signal respiratoire moyen (311) est prévue pour calculer le signal respiratoire moyen (311) du sujet (111) sur la base d'un facteur de sensibilité temporelle (412, 413) et d'un facteur de sensibilité d'amplitude (415, 416) du signal radar (310, 410).

**3.** Dispositif (100) selon la revendication 1,
où l'unité de détection d'événement respiratoire pendant le sommeil (223) est en outre prévue pour détecter :

un premier événement respiratoire pendant le sommeil sur la base d'une valeur (640, 650) correspondant à la valeur seuil ou plus dans la première information de rythme respiratoire pendant le sommeil (511) ; et
un deuxième événement respiratoire pendant le sommeil sur la base d'une valeur (640, 650) atteignant la valeur seuil dans la deuxième information de rythme respiratoire pendant le sommeil (512).

**4.** Dispositif (100) selon l'une des revendications 1 à 3,
où l'unité de génération d'informations de rythme respiratoire pendant le sommeil (222) est en outre prévue pour générer des informations d'état de mouvement sur la respiration pendant le sommeil du sujet (111) par détection d'un cas où le signal radar (310, 410) est supérieur au signal respiratoire moyen (311).

**5.** Dispositif (100) selon la revendication 4,
où l'unité de génération d'informations de rythme respiratoire pendant le sommeil (222) est en outre prévue pour générer :

une première information de schéma de mouvement sur un mouvement pendant le sommeil du sujet (111) sur la base de la continuation ou non des informations d'état de mouvement ; et
une deuxième information de schéma de mouvement pendant le sommeil sur la base de la continuation ou non des informations d'état de mouvement et d'une valeur seuil prédéterminée étant un temps seuil spécifique.

**6.** Dispositif (100) selon la revendication 5,
où l'unité de détection d'événement respiratoire pendant le sommeil (223) est en outre prévue pour détecter :

un premier événement de mouvement pendant le sommeil sur la base d'une valeur (640, 650) correspondant à la valeur seuil ou plus dans la première information de schéma de mouvement pendant le sommeil ; et
un deuxième événement de mouvement pendant le sommeil sur la base d'une valeur (640, 650) atteignant la valeur seuil dans la deuxième information de schéma de mouvement pendant le sommeil.

**7.** Procédé d'analyse de la respiration pendant le sommeil au moyen d'un radar (110), comprenant :

a) la transmission (S1010) d'un signal radar (310, 410) vers un sujet (111) ;
b) la réception (51020) du signal radar (310, 410) réfléchi par le sujet (111) ;
c) le calcul (S1030) d'un signal respiratoire moyen (311) du sujet (111) sur la base du signal radar réfléchi (310, 410) ;
d) la génération (S1040) d'informations de rythme respiratoire pendant le sommeil (511, 512, 610, 620) du sujet (111) par comparaison du signal radar (310, 410) avec le signal respiratoire moyen (311) ; et
e) la détection (S 1050) d'un événement respiratoire pendant le sommeil sur la base des informations de rythme respiratoire pendant le sommeil (511, 512, 610, 620) ;

**caractérisé en ce que** :
f) les informations de rythme respiratoire pendant le sommeil (511, 512, 610, 620) comprennent des informations d'état respiratoire (510, 520) générées sur la respiration pendant le sommeil du sujet (111), comprenant :

g1) la génération d'une première information de rythme respiratoire pendant le sommeil (511) sur la respiration pendant le sommeil du sujet (111) sur la base de la continuation ou non des informations d'état respiratoire (510) ;
g2) la génération d'une deuxième information de rythme respiratoire pendant le sommeil (512) sur la base de la continuation ou non des informations d'état respiratoire (312) et d'une valeur seuil prédéterminée ; et
h) le calcul de la première information de rythme respiratoire pendant le sommeil (511) et de la deuxième information de rythme respiratoire pendant le sommeil (512) pour obtenir une valeur continuant à croître dans le temps pendant que le signal radar (310, 410) est inférieur au signal respiratoire moyen (311) ;
i) où la deuxième information de rythme respiratoire pendant le sommeil (512) est initialisée à la valeur seuil prédéterminée avant de croître à nouveau, et
h) où la valeur seuil prédéterminée est un temps seuil spécifique.

**8.** Procédé d'analyse de la respiration du sommeil selon la revendication 7,
où, lors du calcul du signal respiratoire moyen (311), le signal respiratoire moyen (311) du sujet (111) est calculé sur la base d'un facteur de sensibilité temporelle (412, 413) et d'un facteur de sensibilité d'amplitude (415, 416) du signal radar (310, 410).

**9.** Procédé selon la revendication 7,
où la détection d'un événement respiratoire pendant le sommeil comprend en outre :

la détection d'un premier événement respiratoire pendant le sommeil sur la base d'une valeur (640, 650) correspondant à la valeur seuil ou plus dans la première information de rythme respiratoire pendant le sommeil (511, 512, 610, 620) ; et
la détection d'un deuxième événement respiratoire pendant le sommeil sur la base d'une valeur (640, 650) atteignant la valeur seuil dans la deuxième information de rythme respiratoire pendant le sommeil (511, 512, 610, 620).

**10.** Procédé selon l'une des revendications 7 à 9,
où la génération d'informations de rythme respiratoire pendant le sommeil (511, 512, 610, 620) comprend en outre la génération d'informations d'état de mouvement sur la respiration pendant le sommeil du sujet (111) par détection d'un cas où le signal radar (310, 410) est supérieur au signal respiratoire moyen (311).

**11.** Procédé selon la revendication 10,
où la génération d'informations de rythme respiratoire pendant le sommeil (511, 512, 610, 620) comprend en outre :

la génération d'une première information de schéma de mouvement sur un mouvement pendant le sommeil du sujet (111) sur la base de la continuation ou non des informations d'état de mouvement ; et
la génération d'une deuxième information de schéma de mouvement pendant le sommeil sur la base de la continuation ou non des informations d'état de mouvement et d'une valeur seuil prédéterminée étant un temps seuil spécifique.

**12.** Procédé selon la revendication 11,
où la détection d'un événement respiratoire pendant le sommeil comprend en outre :

la détection d'un premier événement de mouvement pendant le sommeil sur la base d'une valeur (640, 650) correspondant à la valeur seuil ou plus dans la première information de schéma de mouvement pendant le sommeil ; et
la détection d'un deuxième événement de mouvement pendant le sommeil sur la base d'une valeur (640, 650) atteignant la valeur seuil dans la deuxième information de schéma de mouvement pendant le sommeil.

**13.** Programme informatique stocké dans un support d'enregistrement lisible par ordinateur, comprenant une séquence d'instructions pour analyser la respiration pendant le sommeil au moyen d'un radar (110),
où ledit programme informatique comprend une séquence d'instructions dont l'exécution par un dispositif informatique entraîne la mise en oeuvre par le dispositif selon l'une ou plusieurs des revendications 1 à 6 des étapes selon l'une ou plusieurs des revendications 7 à 12.

## FIG. 1

# FIG. 2

*FIG. 3*

## FIG. 4A

## FIG. 4B

EP 4 147 635 B1

*FIG. 5*

FIG. 6

EP 4 147 635 B1

# FIG. 7A

| Classification | | detection item | L | F | T | b0 | b1 | b2 | b3 | b4 |
|---|---|---|---|---|---|---|---|---|---|---|
| 711 | Apnea | Cetral ~711a | 30 | 1.5 | 17.5 | −3.21 | 1.28 | −1.38 | 0.05 | −0.05 |
| | | Mixed ~711b | 20 | 1.75 | 7.5 | −11.05 | 0.00 | 0.02 | 0.96 | −1.02 |
| | | Obstructive ~711c | 30 | 1.75 | 5 | 62.68 | −0.07 | −0.06 | −0.19 | 1.30 |
| 712 | Hypopnea | Cetral | 5 | 0.5 | 0.3 | 0.00 | 0.00 | 0.00 | 0.00 | 0.00 |
| | | Mixed | 5 | 0.5 | 12.5 | −0.02 | 0.00 | 0.97 | 0.00 | 0.00 |
| | | Obstructive | 10 | 1.75 | 7.5 | 14.05 | −0.40 | 0.87 | −0.66 | 0.85 |
| 713 | Arousal | LM(Limb Movement) | 10 | 1.75 | 7.5 | −2.51 | −0.09 | 0.10 | 1.01 | −1.14 |
| | | RERA(Respiratory Effort Relative Arousal) | 40 | 1.75 | 7.5 | −1.45 | 0.07 | −0.07 | −0.03 | −0.07 |
| | | Resp(Respiratory) | 30 | 1.75 | 5 | 29.81 | −0.03 | 0.01 | −0.03 | 1.15 |
| | | Snore | 60 | 1.75 | 1 | 3.10 | 0.00 | −0.01 | −0.02 | 0.02 |
| | | Spontaneous | 20 | 1.75 | 0.5 | −13.56 | 0.00 | −0.01 | 0.01 | 0.01 |

710, 720 (721, 722, 723), 730 (731, 732, 733, 734, 735)

EP 4 147 635 B1

# FIG. 7B

| # | Target Event | # of Events | All | Headboard V30 | Headboard T0 | Ceiling V30 | Ceiling T0 | Ceiling T1 | Optimized |
|---|---|---|---|---|---|---|---|---|---|
| 1 | Apnea | 0 | 0.0000 | 0.0000 | 0.0000 | 0.0000 | 0.0000 | 0.0000 | 0.0000 |
| 2 | Apnea Central | 811 | 0.6452 | 0.9250 | 0.9094 | 0.5603 | 0.5997 | 0.9206 | 0.7946 |
| 3 | Apnea Mixed | 2691 | 0.8912 | 0.9921 | 0.9942 | 0.7986 | 0.8520 | 0.9593 | 0.9551 |
| 4 | Apnea Obstructive | 18364 | 0.6587 | 0.8896 | 0.7320 | 0.7000 | 0.7247 | 0.9316 | 0.7702 |
| 5 | Hypopnea | 0 | 0.0000 | 0.0000 | 0.0000 | 0.0000 | 0.0000 | 0.0000 | 0.0000 |
| 6 | Hypopnea Central | 0 | 0.0000 | 0.0000 | 0.0000 | 0.0000 | 0.0000 | 0.0000 | 0.0000 |
| 7 | Hypopnea Mixed | 8 | 0.2336 | 0.3791 | 0.4355 | 0.5125 | 0.4410 | 0.0000 | 0.4470 |
| 8 | Hypopnea Obstructive | 23440 | 0.4279 | 0.7559 | 0.5517 | 0.4737 | 0.4647 | 0.7707 | 0.5960 |
| 9 | R | 21590 | 0.3776 | 0.6377 | 0.5691 | 0.6447 | 0.6087 | 0.8119 | 0.6429 |
| 10 | W | 32167 | 0.2361 | 0.6709 | 0.6383 | 0.4562 | 0.5312 | 0.7526 | 0.5751 |
| 11 | N1 | 27734 | 0.7171 | 0.9071 | 0.7672 | 0.7675 | 0.8281 | 0.9703 | 0.8282 |
| 12 | N2 | 57991 | 0.3689 | 0.8135 | 0.7419 | 0.7526 | 0.7882 | 0.7786 | 0.7763 |
| 13 | N3 | 20328 | 0.5608 | 0.5793 | 0.5245 | 0.6616 | 0.6096 | 0.7561 | 0.6264 |
| 14 | S4 | 0 | 0.0000 | 0.0000 | 0.0000 | 0.0000 | 0.0000 | 0.0000 | 0.0000 |
| 15 | LM Arousal | 1325 | 0.4324 | 0.4524 | 0.2598 | 0.6012 | 0.6129 | 0.8386 | 0.6215 |
| 16 | RERA | 4574 | 0.3917 | 0.6498 | 0.5740 | 0.6109 | 0.5106 | 0.8326 | 0.6247 |
| 17 | Resp Arousal | 27773 | 0.7570 | 0.9488 | 0.7992 | 0.7564 | 0.6734 | 0.9564 | 0.8175 |
| 18 | Snore Arousal | 3040 | 0.3355 | 0.5166 | 0.5978 | 0.6015 | 0.5615 | 0.6895 | 0.5845 |
| 19 | Spontaneous Arousal | 2987 | 0.4748 | 0.5820 | 0.4448 | 0.6517 | 0.6628 | 0.8316 | 0.6172 |
| 20 | Apnea All | 21866 | 0.7754 | 0.9476 | 0.8175 | 0.7441 | 0.7677 | 0.9294 | 0.8254 |
| 21 | Hypopnea All | 23448 | 0.4279 | 0.7560 | 0.5519 | 0.4739 | 0.4647 | 0.7707 | 0.5962 |
| 22 | Araousal All | 39699 | 0.7089 | 0.9184 | 0.7948 | 0.7683 | 0.7016 | 0.9720 | 0.8143 |
| 23 | Apnea + Hypopnea | 45314 | 0.6759 | 0.8858 | 0.7149 | 0.7358 | 0.6660 | 0.9317 | 0.7730 |
| 24 | Apnea + Hypopnea + Wake | 77481 | 0.6637 | 0.9431 | 0.7610 | 0.7514 | 0.7336 | 0.8809 | 0.8058 |
| 25 | Apnea + Hypopnea + Araousal | 85013 | 0.7263 | 0.9389 | 0.7665 | 0.7676 | 0.7191 | 0.9601 | 0.8164 |
| 26 | Apnea + Hypopnea + Araousal + Wake | 117180 | 0.7246 | 0.9558 | 0.7830 | 0.7977 | 0.7735 | 0.9403 | 0.8386 |
| 27 | Central | 811 | 0.6452 | 0.9250 | 0.9094 | 0.5603 | 0.5997 | 0.9206 | 0.7946 |
| 28 | Mixed | 2699 | 0.8912 | 0.9921 | 0.9943 | 0.7986 | 0.8526 | 0.9593 | 0.9552 |
| 29 | Obstuctive | 41804 | 0.6133 | 0.8554 | 0.6973 | 0.6794 | 0.5981 | 0.9293 | 0.7327 |
| 30 | Apnea + Hypopnea + RERA | 49888 | 0.6694 | 0.8858 | 0.7138 | 0.7365 | 0.6760 | 0.9362 | 0.7734 |
| 31 | Position-Supine | 117543 | 0.2923 | 0.7593 | 0.6618 | 0.7712 | 0.7783 | 0.8072 | 0.7617 |
| 32 | Position-Prone | 141 | 0.2326 | 0.2888 | 0.3536 | 0.3237 | 0.3579 | 0.7852 | 0.3567 |
| 33 | Position-Left | 14134 | 0.1983 | 0.2682 | 0.3400 | 0.4825 | 0.5559 | 0.6829 | 0.4540 |
| 34 | Position-Right | 22005 | 0.3387 | 0.5968 | 0.5981 | 0.5802 | 0.4313 | 0.5796 | 0.5658 |
| 35 | Position-Upright | 0 | 0.0000 | 0.0000 | 0.0000 | 0.0000 | 0.0000 | 0.0000 | 0.0000 |
| 36 | Position-Left + Position-Right | 36139 | 0.3243 | 0.5077 | 0.4350 | 0.6210 | 0.5223 | 0.6104 | 0.5398 |
| | Population | | 219 | 43 | 43 | 57 | 60 | 24 | 227 |

740

750 751 752 753 754

# FIG. 8A

# FIG. 8B

# FIG. 8C

*FIG. 9*

| | | 920 | 930 | 940 | 950 |
|---|---|---|---|---|---|
| | | Value | AD | Control | Prediction |
| 911 | TST | 183 ⁓921 | 0.8692 ⁓931 | 0.1308 ⁓941 | AD ⁓951 |
| 912 | AHI | 10.49 ⁓922 | 0.2179 ⁓932 | 0.7821 ⁓942 | Control ⁓952 |
| 913 | RDI | 14.43 ⁓923 | 0.9801 ⁓933 | 0.0199 ⁓943 | AD ⁓953 |

910 { 911, 912, 913 }

[000171]

## FIG. 10

```
                    ┌──────────┐
                    │  start   │
                    └──────────┘
                         │
                         ▼
        ┌─────────────────────────────────────┐
        │   Transmit radar signal toward subject │ ──── S1010
        └─────────────────────────────────────┘
                         │
                         ▼
        ┌─────────────────────────────────────┐
        │  receive radar signal reflected from subject │ ──── S1020
        └─────────────────────────────────────┘
                         │
                         ▼
        ┌─────────────────────────────────────┐
        │   calculate average breathing signal of │ ──── S1030
        │    subject based on radar signal        │
        └─────────────────────────────────────┘
                         │
                         ▼
        ┌─────────────────────────────────────┐
        │ generate sleep breathing pattern information of │ ──── S1040
        │  subject by comparing radar signal with          │
        │     average breathing signal and                 │
        └─────────────────────────────────────┘
                         │
                         ▼
        ┌─────────────────────────────────────┐
        │   detect sleep breathing event based on │ ──── S1050
        │    sleep breathing pattern information   │
        └─────────────────────────────────────┘
                         │
                         ▼
                    ┌──────────┐
                    │   end    │
                    └──────────┘
```

[000172]

32

# FIG. 11

```
            ┌─────────────┐
            │    start    │
            └──────┬──────┘
                   │
                   ▼
   ┌──────────────────────────────────┐
   │  transmit radar signal toward     │──S1110
   │           subject                 │
   └───────────────┬──────────────────┘
                   │
                   ▼
   ┌──────────────────────────────────┐
   │  receive radar signal reflected   │──S1120
   │          from subject             │
   └───────────────┬──────────────────┘
                   │
                   ▼
   ┌──────────────────────────────────┐
   │  derive sleep time information of │
   │  subject and event occurrence     │──S1130
   │  information for sleep items      │
   │  based on radar signal            │
   └───────────────┬──────────────────┘
                   │
                   ▼
   ┌──────────────────────────────────┐
   │  calculate breathing-related      │
   │  index information based on sleep │──S1140
   │  time information and event       │
   │  occurrence information for       │
   │  sleep items                      │
   └───────────────┬──────────────────┘
                   │
                   ▼
   ┌──────────────────────────────────┐
   │  determine whether subject has    │
   │  sleep-related disease based on   │──S1150
   │  breathing-related index          │
   │  information                      │
   └───────────────┬──────────────────┘
                   │
                   ▼
            ┌─────────────┐
            │     end     │
            └─────────────┘
```

## REFERENCES CITED IN THE DESCRIPTION

**Patent documents cited in the description**

- KR 102321991

- KR 1020180077453

**Non-patent literature cited in the description**

- **WANG QISONG et al.** Frequency-Modulated Continuous Wave Radar Respiratory Pattern Detection Technology Based on Multifeature. *JOURNAL OF HEALTHCARE ENGINEERING,* 10 August 2021, vol. 2021, 1-18